(19) Europäisches Patentamt
European Patent Office
Office européen des brevets

(11) Publication number: **0 234 500**
**A1**

(12) # EUROPEAN PATENT APPLICATION

(21) Application number: 87102321.4

(22) Date of filing: 18.02.87

(51) Int. Cl.$^3$: A 61 K 9/12
A 61 K 31/47

(30) Priority: 18.02.86 US 830421

(43) Date of publication of application: 02.09.87 Bulletin 87/36

(84) Designated Contracting States: AT BE CH DE ES FR GB GR IT LI LU NL SE

(71) Applicant: RORER PHARMACEUTICAL CORPORATION
500 Virginia Drive
Fort Washington Pennsylvania(US)

(72) Inventor: Coutts, Stephen M.
61 Walworth Avenue
Scarsdale New York(US)

(72) Inventor: Dietchweiler, Ralph L.
38 Orchard Street
Cos Cob Connecticut(US)

(74) Representative: Patentanwälte Grünecker, Kinkeldey, Stockmair & Partner
Maximilianstrasse 58
D-8000 München 22(DE)

(54) **Antiasthma inhalation compositions.**

(57) Compositions of matter to be dispersed from drug delivery pressure devices to the oronasal passageways of a patient for the treatment of asthmatic conditions, the composition being dissolved in ethyl alcohol and admixed with an acceptable propellant.

EP 0 234 500 A1

## ANTIASTHMA INHALATION COMPOSITIONS

This invention relates to compositions possessing potent activity for the prophylactic and therapeutic treatment of bronchospasm or asthmatic attack.

Antigen-antibody interaction in mammals manifest themselves as alergic reactions, such as allergic rhinitis, hay fever, and bronchial asthma. The latter is one of the most debilating of the allergic reactions and results in bronchospasm, paroxysm and a general constriction of airway passages. One class of medications designed to relieve constriction of airflow is bronchodilators and provide symptomatic relief. In contrast, another class of medications prevent the release of mediators of anaphylaxis thereby preventing elicitation of bronchoconstriction.

The present invention relates to the latter-mentioned class of medications.

Compounds having activity in regulating the formation of lipoxygenases have been known for some time and have been disclosed as having value in the treatment of inflammatory conditions and allergic responses such as anaphylaxis and asthma.

Lipoxygenases in mammals have been found in the lung, platelets, and white cells. They are enzymes capable of oxidizing arachidonic acid into hydroperoxyeicosatetraenoic acids (HPETEs) and their stable products hydroxyeicosatetraenoic acids (HETEs). Lipoxygenases are classified according to the position in the arachidonic acid which is oxygenated. Platelets metabolize arachidonic acid to 12-HPETE, while polymorphonuclear

leukocytes contain 5 and 15 lipoxygenases. It is known that 12-HPETE and 5, 12-diHETE (also denoted as $LTB_4$) are chemotactic for human neutrophils and eosinophils, and may augment the inflammation process. 5-HPETE is known to be a precursor of slow-reacting substance of anaphylaxis (SRS-A). The SRS family of molecules, such as leukotrienes B, C, and D, have been shown to be potent bronchoconstrictors (see, NATURE 288, 484-486 (1980)).

Compounds for the treatment of inflammatory conditions and allergic responses can be administered to the host in a variety of forms adapted to the chosen route of administration, i.e., orally, intravenously, intramuscularly or subcutaneously.

For the treatment of asthmatic conditions the inhalation route of application appears to be eminently suitable: epithelial mast cells in the lung are the key cells to be stabilized by pharmacological intervention and these cells are more effectively and rapidly reachable by an aerosol preparation than by other routes of administration; and administration of an antiasthmatic drug directly into the lung in an aerosol formulation would overcome the first pass metabolization by the liver associated by other routes of administration.

Accordingly, the present invention provides antiasthma formulations in aerosol form for the prophylactic and therapeutic treatment of asthma.

The present invention utilizes compounds known for antiasthma activity. Such compounds are disclosed, for example, in South African Patent No. 83/8926 assigned to USV Pharamaceutical Corporation.

According to the invention there is provided an aerosol formulation for inhalation for the treatment of asthmatic conditions comprising an effective amount of an antiasthma compound dissolved in a pharmaceutically acceptable alcohol and admixed with a pharmaceutically acceptable propellant or propellant mixture.

More particularly, the present invention relates to a composition of matter to be dispersed from a drug delivery pressure device for the treatment of asthmatic conditions in a mammal comprising an effective amount of an antiasthmatic compound selected from 2-(3-(1-hydroxyhexyl)phenoxymethyl)quinoline hydrochloride, 2-(3-hexanoylphenoxymethyl)quinoline, 2-(3-(2-(2-hydroxy)heptyl)phenyloxymethyl)quinoline hydrochloride, 2-[3-(1-hydroxy-2-methylhexyl)phenoxymethyl]quinoline, 2-(3-(6-phenoxy-1-hydroxyhexyl)-phenoxymethyl)quinoline, 2-[3-(6-phenoxy-1-hydroxy-1-methylhexyl)phenoxymethyl]quinoline, 2-[3-(1-hydroxy-2,2-dimethylhexyl)phenoxymethyl]quinoline, 2-(3-(1,2,2--trimethyl-1-hydroxyhexyl)phenoxymethyl)quinoline, 2-(3-(1-hydroxypentyl)phenoxymethyl)quinoline, $\alpha$ -(3-2-quinolinylmethoxyphenyl)hexyl 2,2-dimethyl propanoate, 2-(3-(6- phenoxy-1,2,2-trimethyl-1-hydroxyhexyl)-phenoxymethyl)quinoline, 2-(3-(1-hydroxy-6,6,6-trilfuoro-hexyl)phenoxymethyl)quinoline, or 2-(3-(5,5,5-trifluoro-1-methyl-1-hydroxypentyl)phenoxymethyl)quinoline or

mixtures thereof dissolved in ethyl alcohol and admixed with a sufficient amount of a pharmaceutically acceptable propellant capable of effective transfer of said antiasthmatic compound from said drug delivery pressure device to the oronasal passageways of a patient.

The preferred pharmaceutically accpetable alcohol is ethanol having no more than 5 per cent water therein.

The preferred propellant is a mixture of dichlorodifluoromethane (P-12) and dichlorotetrafluoroethane (P-114).

In accordance with the present invention, antiasthmatic aerosol inhalation compositions are provided comprising:

(a) 0.25% to 20% w/w of an antiasthma active compound solubilized in

(b) 2.5% to 30% w/w ethyl alcohol and

(c) admixed with a propellant mixture of 15% to 50%, and more preferably, 40% to 50% w/w propellant 12 and 30% to 50% and more preferably, 40% to 50% w/w propellant 114, said propellant mixture to constitute about 50% to 98% w/w of the total composition.

In another embodiment of the present invention, suspension formulations are provided comprising: 0.25% -20% w/w of a micronized antiasthma active compound suspended in a mixture of a propellant and a pharamaceutically acceptable surface active agent, said mixture to constitute about 50% - 98% w/w of the total composition. Preferred pharmaceutically acceptable surface active agents include

| Range Percent | Ingredient |
|---|---|
| 0.1 to 2.5% | Oleyl Alcohol |
| 0.1 to 2.5% | Oleic Acid |
| 0.5 to 5.0% | Lecithin |
| 0.1 to 2.5% | Tween 80 |

(Tween 80 is a mixture of oleate esters of sorbitol and sorbitol anhydrides having the formula

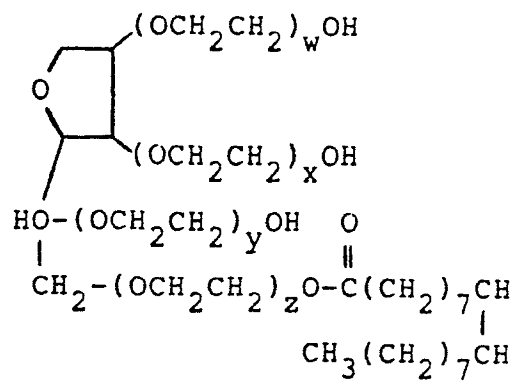

where w + x + y + z has an average value of 20.)

Preferred propellants for the purpose of the present invention include: Propellant 11 (trichloromonofluoromethane), Propellant 12, Propellant 114 or mixtures thereof.

The compounds utilized in the present invention may be made by standard organic reactions utilizing starting materials that are either commercially available or which may be synthesized using art-recognized synthetic procedures. The preferred synthesis of the compounds are described hereunder.

EXAMPLE 1

2-(3-(1-Hydroxyhexyl)phenoxymethyl)quinoline hydrochloride

A suspension of 2-chloromethylquinoline hydrochloride (15 g, 0.07 mol), 3-(1-hydroxyhexyl)phenol (14 g, 0.072 mol), finely powdered anhydrous potassium carbonate (22.5 g, 0.16 mol), cesium carbonate (4.7 g, 0.014 mol) and sodium iodide (2.2 g, 0.015 mol) in dry acetone (200 ml) was refluxed overnight (ca. 15 hr.). The reaction mixture was then poured into water (500 ml), and the aqueous solution was thoroughly extracted with ethyl acetate. The organic extract was washed with 1N NaOH solution, water and brine. After drying over $MgSO_4$, all voltailes were removed from the organic extract to leave the crude product as a light brown viscous liquid. This was dissolved in dry ether, and then treated with an ethereal solution of HCl. A gummy solid precipitated which was washed with ether to remove excess of HCl, and then triturated with ether-petroleum ether (1:1). The gummy solid turned into a free-flowing powder weighing 22.6 g, which was crystallized from ethanol-hexanes to yield the pure product as the hydrochloride salt as an off-white solid, mp 118-119°C.

The free base can be also purified. The crude product which was a light brown liquid (before treatment with ethereal HCl) can be crystallized from methanol-hexanes to yield the free base as a white solid, m.p. 67-69°C.

EXAMPLE 2

2-(3-Hexanoylphenoxymethyl)quinoline

A solution of 1-(3-(hydroxyphenyl)-1-hexanol (3 g) in methylene chloride (200 ml) was added to a well-stirred suspension of pyridinium chlorochromate (5.1 g) and sodium acetate (2.5 g) in methylene chloride (150 ml). The mixture was stirred at room temperature for two hours. Ether (100 ml) was added, and the brown granular precipitate was removed by filtration. All volatiles were removed from the filtrate, and the residual liquid was purified by chromatography on silica gel using ether as eluent. The desired ketone, 3-hexanoylphenol, was isolated as a clear, colorless oil.

This ketone (1.2 g) was refluxed with 2-chloromethylquinoline hydrochloride (1.3 g), potassium carbonate (8.6 g), potassium iodide (0.05 g) and cesium carbonate (0.05 g) in acetone (300 ml) for 24 hours. The reaction mixture was filtered, and the filtrate was concentrated, dissolved in chloroform, and then washed with sodium hydroxide solution (5%), water and brine. After drying this solution over $MgSO_4$, all volatiles were removed. The residue was purified by chromatography on silica gel (25% ethyl acetate in hexanes). The pure desired product was isolated as a tan solid, m.p. 52-55°C.

EXAMPLE 3

2-(3-(2-(2-Hydroxy)heptyl)phenoxymethyl)quinoline hydrochloride

A mixture of 2-chloromethyl quinoline hydrochloride (3.1 g) and 2-(3-hydroxyphenyl)-2-heptanol (3 g) in acetone (300 ml) containing potassium carbonate (20 g) and potassium iodide (0.2 g) was refluxed overnight. The reaction mixture was cooled, and most of the volatiles were removed at the rotary evaporator. The residue was taken up in ethyl acetate, and was washed with sodium hydroxide solution (5%) and brine. The organic layer was dried over magnesium sulfate and all solvent was removed to leave the crude product (weighing about 6 g), which was purified by chromatography (silica gel; 20% ethyl acetate in hexane). The free base, isolated as an oil, was dissolved in dry ether, and treated with hydrogen chloride gas to give the pure desired salt as a white solid, m.p. 125°C. (dec.).

EXAMPLE 4

2-[3-(1-Hydroxy-2-methylhexyl)phenyloxymethyl]quinoline

A. A solution of the ketone, 2-(3-hexanoyl-phenoxymethyl)-quinoline (prepared from 3-hexanoyl-phenoxymethyl)-quinoline (prepared from 3-(hexanoylphenol and 2-chloromethyl quinoline) (6 g, 0.018 mol) in dry THF (25 ml) was added slowly to a mildly refluxing suspension of sodium hydride (1.3 g, 0.054 mol) in dry THF (75 ml) containing excess methyl iodide (10.3 g, 0.073 mol). After the addition of the ketone was complete (30 min.), the reaction mixture was refluxed for 4 hours. After cooling the mixture to room temperature, excess NaH was destroyed by adding methanol. All volatiles were removed at the rotary evaporator and the residue was taken up in ethyl acetate. The organic extract was washed with water and brine, and dried over anhydrous magnesium sulfate. Upon removal of all solvent, the mono-methylated ketone (2-[3-(2-methyl-hexanoyl)phenoxy- methyl]quinoline, was obtained as a yellow oil weighing 8 g.

B. This ketone (8 g) was next reduced with sodium borohydride (1.4 g, 0.036 mol) in ethanol (100 ml) at room temperature for about 15 hours. A little water and methanol was added to the reaction mixture and then most of the volatiles were removed at the rotary evaporator. The residue was thoroughly extracted with ethyl acetate, and the organics were washed with water, brine and then dried over anhydrous $MgSO_4$. All solvent was removed to leave a yellow oil (6 g) which was chromatographed on silica gel using 20% ethyl acetate

in hexane as eluent. The desired mono methylated alcohol was obtained as a yellowish solid (1.5 g), m.p. 64-67°C.

EXAMPLE 5

2-(3-(6-Phenoxy-1-Hydroxyhexyl)Phenoxymethyl)Quinoline

A. Synthesis of 5-phenoxypentyl bromide

To a well-stirred suspension of sodium hydride (15.3 g, 0.32 mol; 50% oil dispersion) in dry DMF (500 ml) containing phenol (25 g, 0.26 mol), was added dropwise 1,5-dibromopentane (122.2 g, 0.53 mol). After the addition was complete, the reaction mixture was stirred at 60-70°C for 4 hours. Most of the DMF was next removed under reduced pressure, and the residue was dissolved in ethyl acetate (250 ml). The organic extract was washed successively with a 5% sodium hydroxide solution, water and brine. After drying the organics over anhydrous magnesium sulfate, all volatiles were removed. The resisual yellow liquid was then distilled under reduced pressure, and the pure product was collected at 118-125°C/1 mm as a clear colorless liquid (26 g, 40% yield).

B. Synthesis of 3-(6-phenoxy-1-hydroxyhexyl)phenol

To gently refluxing dry tetrahydrofuran (70 ml) containing magnesium turnings (1.1 g, 44 mmol) and a small crystal of iodine, was added dropwise from a dropping funnel a solution of 5-phenoxypentyl bromide (11 g, 44 mmol) in dry THF (50 ml). After about 5-7 minutes, the Grignard reaction started as indicated by the disappearance of the brown iodine color. The heating was adjusted so that the solution refluxed gently. After the addition of the halide was complete (ca. 30 min.), the clear solution was refluxed for about 1 hour. This solution of the Grignard reagent was cooled to room temperature.

A solution of 3-hydroxybenzaldehyde (5.0 g, 41 mmol) in dry THF (50 ML) was added dropwise to a well-stirred suspension of sodium hydride (2.5 g, 52 mmol; 50% oil dispersion) in dry THF (50 ml) at room temperature. The temperature was not allowed to go above ca. 40°C. After the addition of 3-hydroxybenzaldehyde was complete (ca. 30 min.) the slightly cloudy solution was warmed to 45-50°C and stirred for 1 hour at this temperature. The resulting clear solution of sodium 3-formyl phenoxide was cooled to room temperature and added dropwise using a cannula, to the well-stirred solution of the Grignard reagent. A thick precipitate immediately formed. This mixture was maintained at 4-45°C during the addition of the sodium salt. After the addition was complete (ca. 2 hours), the reaction mixture was stirred at this temperature for another hour, and then cooled in an ice bath. The excess reactive materials (sodium hydride, Grignard reagent, etc.) were destroyed slowly by carefully adding methanol (30 ml), and then a saturated ammonium chloride solution (350 ml) was added to dissolve all solids. The organic layer was separated, and the aqueous layer was extracted with ether (2 x 75 ml). The combined organics were washed with water, brine, and then dried over anhydrous magnesium sulfate. After drying the extract, all volatiles were removed to obtain a dark liquid. This oil was triturated with a 1:1 mixture of ether and petroleum ether (35-55°C) when an off-white solid was obtained (12 g). This was crystallized from methanol-ethyl acetate to give a white solid, m.p. 148-9°C.

C. Synthesis of 2-(3-(6-phenoxy-1-hydroxyhexyl) phenoxy-methyl)quinoline

A mixture of 2-chloromethyl quinoline (1.1 g; 6.3 mmol), 3-(6-phenoxy-1-hydroxyhexyl)phenol (1.8 g, 6.3 mmol) and finely powdered anhydrous potassium carbonate (1.74 g, 12.6 mmol) in acetone (40 ml) containing catalytic amounts of finely powdered, anhydrous cesium carbonate (0.4 g, 1.3 mmol) and sodium iodide (0.05 g, 0.33 mmol) was refluxed overnight (15 hours). The reaction mixture was cooled to the room temperature and filtered. The solid residue was washed thoroughly with acetone (3 x 15 ml), and all the filtrates were combined. All volatiles were removed from the combined acetone filtrate to leave a light yellow viscous liquid which solidified to an off-white solid on trituration with a 1:1 mixture of ether and petroleum ether (35-55°C). This solid was chromatographed on silica gel with 35% ethyl acetate in hexanes to yield the desired product (1.6 g, 60%) as a white crystalline solid, m.p. 83-84.5°C.

## EXAMPLE 6

## 2-[3-(6-Phenoxy-1-Hydroxy-1-Methylhexyl)Phenoxymethyl]Quinoline

To a gently refluxing dry tetrahydrofuran (100 ml) containing magnesium turnings (0.5 g, 21 mmol) and a small crystal of iodine, was added dropwise pure 5-phenoxypentyl bromide (5 g, 21 mmol; b.p. 118-125°C/1 mm). After a few minutes, the reaction started, and the iodine color disappeared. The addition of the bromide continued at such a rate as to maintain a gentle reflux. After the addition was complete (ca. 20 min.), the solution was refluxed for another 30 min., and then cooled. This cold Grignard solution was next added dropwise via a cannula to an ice cold solution of 3-(2-quinolinylmethoxy)-acetophenone (5 g, 18 mmol) in dry THF (100 ml). After the addition was complete (ca. 45 min.), the ice bath was removed, and the solution was slowly heated to mild reflux for 3 hours. (The reaction was rather slow at room temperature.) The reaction mixture was cooled in an ice bath, and a saturated ammonium chloride solution was added to it. The organic layer was separated, washed with brine, and dried over anhydrous magnesium sulfate. All volatiles were removed to give a crude oily reaction mixture which was purified by chromatography (HPLC on silica gel, 20% ethyl acetate in hexanes used as eluent). The desired alcohol (Rf 0.2) was obtained as a pale yellow liquid (2.6 g, 33%).

EXAMPLE 7

2-[3-(1-Hydroxy-2,2-Dimethylhexyl)Phenoxymethyl]Quinoline

A. Synthesis of 3-(2,2-dimethyl hexanoly)phenol

The benzyl ether of the desired phenol (26 g) was taken up in methanol (150 ml) and this solution was hydrogenated in a Parr apparatus under an atmosphere of hydrogen (45-50 lbs./sq.in.) in the presence of 10% palladium on charcoal (10 g). After 20-24 hours, the suspension was filtered on celite; all methanol was removed to obtain the desired phenol in about 83% yield (15.4 g) as a colorless liquid.

The benzyl ether starting compound was prepared by oxidation of 3-(1-hydroxyhexyl)phenyl benzyl ether to the corresponding ketone followed by alkylation with methyl iodide to form the 2,2-dimethyl compound.

B. Preparation of 2-(3-(2,2-dimethylhexanoyl)phenoxymethyl)quinoline:

A mixture of 2-Chloromethylquinoline (12.1 g), 3-(2,2-dimethylhexanoyl-phenol (15 g), finely powdered anhydrous potassium carbonate (20 g) and potassium iodide (0.05 g) in dry acetone (300 ml) was refluxed overnight. The suspension was filtered, and most of the volatiles were removed at the rotary evaporator. The residue was taken up in ethyl acetate, washed with 5% NaOH solution, water, brine and the extract was dried. On removal of all solvent, the crude ether was obtained as a yellow oil. This oil was purified on HPLC using 10% ethyl acetate in hexane as eluent. The pure material was isolated in about 48% yield (12 g) as a light yellow oil.

C. The ketone 2-(3-(2,2-dimethylhexanoyl) phenoxymethyl)quinoline (4 g) was treated with sodium borohydride (0.9 g) in ethanol (70 ml) at room temperature for about 15 hours. The solution was cooled in an ice bath, and excess of borohydride was destroyed by adding cold dilute HCl solution dropwise. After all the reducing agent was destroyed, the pH of the solution was adjusted to about 5-6, and then most of the volatiles were removed. The residue was taken up in ethyl acetate, and the organics were washed with water, brine and dried over $MgSO_4$. The crude alcohol was isolated by removing all solvents. The pure alcohol (1.6 g, 114-116°C.,) was isolated by chromatography on silica gel using 20% ethyl acetate in hexane as eluent.

EXAMPLE 8

2-(3-(1,2,2-Trimethyl-1-Hydroxyhexyl)Phenoxymethyl)Quinoline

The ketone, 2-(3-(2,2-dimethylhexanoyl) phenoxymethyl)quinoline (4.0 g; synthesis of this compound has been described in Example 7B) was dissolved in dry THF (100 ml), and was treated with an ethereal solution of methyl magnesium bromide (4.3 ml, 3.1 M solution). The clear solution was stirred at room temperature overnight. A saturated solution of ammonium chloride was added to the reaction mixture after a precipitate was formed. The solution was filtered, and the filtrate was concentrated to obtain the crude tertiary alcohol, which was chromatographed on silica gel using 20% ethyl acetate in hexanes as eluent to yield the desired compound (0.85 g) as a pale yellow liquid.

EXAMPLE 9

2-(3-(1-Hydroxypentyl)Phenoxymethyl)Quinoline

A mixture of 2-chloromethyl quinoline (5.9 g), 3-(1-hydroxypentyl)phenol (6.7 g) in acetone (200 ml) containing 2N NaOH solution (20.5 ml) was refluxed for three hours. Most of the volatiles were removed at the rotary evaporator, and the residual brown oil was dissolved in ethyl acetate. The organics were washed with 5% NaOH solution and brine. After drying the extracts over $MgSO_4$, all volatiles were removed to leave the crude product. This was purified by chromatography on silica gel using 20% ethyl acetate in hexanes. The pure product was isolated as a light yellow solid. m.p. 63-66°C; 4.4 g (41%).

EXAMPLE 10

α-(3-2-Quinolinylmethoxyphenyl)Hexyl 2,2-Dimethyl Propanoate

To a solution of 2-(3-(1-hydroxyhexyl) phenoxymethyl)quinoline (5 g, 0.015 mol) in $CH_2Cl_2$ (30 ml) containing dry pyridine (1.8 ml) at 0°C, was added a solution of pivaloyl chloride (2 g, 0.0166 mol) in $CH_2Cl_2$ (5 ml) dropwise. After the addition was complete (ca. 10 min.), the solution was stirred at 0°C for 45 min, and then at room temperature for 5 hours. The reaction mixture was poured into water, the organic layer was separated and washed with 1N HCl solution, saturated $NaHCO_3$ solution and brine. After drying the organics over $MgSO_4$, all volatiles were removed. The crude product was chromatographed on silica gel using 10% ethyl acetate in hexanes as eluent to obtain 3.5 g (56%) of pure ester as a faint yellow liquid.

## EXAMPLE 11

**2-(3-(6-Phenoxy-1,2,2-Trimethyl-1-Hydroxyhexyl)Phenoxymethyl) Quinoline;**

To a solution of the ketone, 2-(3-(6-phenoxy-2,2-dimethyl hexanoyl)phenoxymethyl) quinoline (3.6 g) in dry ether (100 ml) at 0°C, was added dropwise, an ethereal solution of methyl magnesium bromide (7.7 ml, 23.8 mmol, 3 eqv.) under an atmosphere of nitrogen. The clear orange solution was stirred overnight at room temperature. To this solution, a saturated ammonium chloride solution was added when the magnesium salts precipitated. The ether layer was separated, washed with water and brine, and then dried over $MgSO_4$. All volatiles were removed to obtain the crude product (4.2 g). This was purified by chromatography on silica gel (25% ethyl acetate in hexanes, 20% ethyl acetate in hexanes and finally 10% ethyl acetate in hexanes) to obtain the pure product as a yellow liquid (0.9 g).

EXAMPLE 12

2-(3-(1-Hydroxy-6,6,6-Triflourohexyl)Phenoxymethyl)Quinoline

To a solution of sodium 3-formylphenolate (prepared from 3-Hydroxybenzaldehyde (14.8 g, 0.121 mol) and sodium hydride (3.0 g, 0.121 mol)) in dry THF (200 ml) was added dropwise a solution of 5,5,5-triflouropentyl magnesium bromide (25 g, 0.122 mol) and magnesium turnings (2.96 g, 0.122 mol) in THF (100 ml) under vigorous stirring. After the addition of the Grignard reagent was complete (20 min.), the reaction mixture was warmed to about 50°C, and stirred at this temperature for about 3 hours. The mixture was cooled in an ice bath, and quenched with a saturated solution of ammonium chloride. The organic layer was separated, washed with brine, and dried over magnesium sulfate. All volatiles were removed to leave the crude desired phenol (21 g) which was pure enough to be used without further purification.

This crude phenol (4.3 g) and 2-chloromethylquinoline (2.86 g) were dissolved in DMSO (75 ml), and then 2N sodium hydroxide solution (8.65 ml) was added. The reaction mixture was stirred at room temperature overnight, poured into water, and then the aqueous solution was extracted with ethyl acetate (4 x 75 ml). The organic extract was washed with 1N NaOH solution, water and brine. After drying the organics over $MgSO_4$, all volatiles were removed to obtain a dark liquid, which was chromatographed on silica gel (5% ethanol in toluene). The pure material was isolated as a beige solid (3 g), m.g. 84.5-86°C.

0234500

## EXAMPLE 13

## 2-(3-(5,5,5-Triflouro-1-Methyl-1-Hydroxypentyl)Phenoxymethyl) Quinoline

A. 3-(2-Quinolinylmethoxy)acetophenone

A mixture of 2-chloromethyl quinoline (25 g, 0.14 mol), 3-hydroxyacetophenone (21.1 g, 0.155 mol), finely powdered ahhydrous potassium carbonate (30 g, 0.22 mol) and a catalytic amount of dry powdered potassium iodide (1 g) in acetone (300 ml) was refluxed overnight. The solution was filtered and the residue was washed with acetone (2 x 25 ml). All volatiles were removed from the combined filtrate to leave the crude oily reaction mixture. This oil was taken up in ethyl acetate (150 ml) and the organic extract was washed with a 5% sodium hydroxide solution (3 x 30 ml). The ethyl acetate solution was washed with water, brine and then dried. On removal of all volatiles, a light yellow liquid was obtained, which on trituration with petroleum ether (35-55°C) gave a beige solid (37 g, 95% yield) m.p. 93-95°C, which was the desired product, pure enough to be used in the next step.

B. 2-(3-(5,5,5-Trifouro-1-methyl-1-hydroxypentyl) phenoxy-methyl-quinoline

The Grignard reagent was prepared in the usual way from 1,1,1-triflouro-4-bromobutane (10 g) and Mg turnings (1.5 g) in dry THF (50 ml). To this was added the above ketone, 3-(2-quinolinylmethoxy) acetophenone (12.1 g) in dry THF (150 ml) dropwise. The solution was stirred overnight (ca. 15 hours), and then a saturated solution of ammonium chloride was added to the reaction mixture. The precipitate was filtered off, and the organic layer was separated, washed with brine and dried over $MgSO_4$. All volatiles were removed to leave a black liquid which was

chromatographed on silica gel to yield 2.3 g of pure product as a light yellow solid, m.p. 71-74°C.

Illustrative formulations of the present invention are shown in Table I, where all percentages are expressed on a weight/weight basis.

TABLE I

| | | | | | | | | | |
|---|---|---|---|---|---|---|---|---|---|
| Compound of Example 1 | 1% | 1% | 2.5% | 3.0% | 3.5% | 5% | 6.0% | 5.0% | 9.0% |
| Anhydrous Ethanol | 5% | 20% | 10.0% | 10.0% | 10.0% | 30% | 20.0% | 30.0% | 30.0% |
| Propellant 114 | 47% | 35% | 43.75% | 43.5% | 43.25% | 15% | 37.0% | 32.5% | 17.0% |
| Propellant 12 | 47% | 44% | 43.75% | 43.5% | 43.25% | 50% | 37.0% | 32.5% | 44.0% |

The formulations of the present invention were found to be stable upon extended aging.

Apparatuses for administering drugs to pulmonary tissue are known in the prior art. These apparatuses have in common a pressurizable inhaler or spray bottle to contain an aerosol propellant and a medication to be applied to the pulmonary tissue. A special kind of delivery device, the nebulizer, is designed to deliver a predetermined dosage of medication to the oronasal cavity of a patient. The present invention contemplates the utilization of these devices, such as bottles and cans, for delivery to the patent of the instant antiasthma formulations.

The containers are equipped with a metered valve of 50 to 150 microliters and a actuator having a suitable orifice to deliver the appropriate size range of the formulation.

In accordance with another aspect of the present invention, there is provided a method of administering an antiasthma drug through an oronasal passage for absorption on the mucous tissue of a patient which comprises the step of: introducing an aerosol formulation of said antiasthmatic drug, by means of pressurized aerosol dispenser/inhaler.

The physician will determine the dosage of the present therapeutic/prophylactic formulations which will be most suitable and it will vary with the particular active compound contained in the formulation chosen, and also, it will vary with the particular patient under treatment. He will generally wish to initiate with small dosages substantially less than the optimum dose of the compound and increase the dosages by small increments until the optimum effect under the circumstances is reached.

The therapeutic dosage will generally be from about 10 to about 1500 mg per day and higher and preferably from about 10 to 600 mg per day or at about 0.2 to 12 mg/kg of body weight in single or divided doses.

Other aspects of the present invention include solubility of the active compound in the pharmaceutically acceptable solvent, ethyl alcohol, the maintainance in solution of the active compound upon the addition of the propellant(s) and the particle size distribution of the aerosol preparation.

The compounds of the present invention are soluble in ethyl alcohol. Illustrative solubilities are shown in Table II.

TABLE II

Compound of Example 1

| Ethanol (W/W) | Solubility at 24°C | Propellants 114/12 (50/50 Mixture) |
|---|---|---|
| 5% | 1.8% | 93.2% |
| 10% | 4.0% | 86.0% |
| 20% | 6.0% | 74.0% |
| 30% | 9.0% | 61.0% |

While amounts of 20% w/w or more of the active compounds can be dissolved in ethyl alcohol, such amounts

are not desirable because of precipitation on addition of the propellant(s) or precipitation on extended storage. Furthermore, particle size distribution is greatly affected by the amounts of the active compounds and ethyl alcohol present in the aerosol formulations. Accordingly, we prefer to use compositions containing 0.25% to 10% w/w of the active compounds in 5% to 20% w/w ethyl alcohol in our aerosol formulations.

Other essential properties of aerosol formulations are the particle size distribution and the related mass median aerodynamic diameter of the particles. It is generally required that the effective range for inahalation products be approximately in the range of 1 to 8 micrometers (um). The particle size distribution helps, inter alia, to reduce loss on impaction on the tongue and oropharynx of the particles expelled from the pressurized container. Illustrative particle size distribution and mass mean aerodynamic diameter (MMAD) are shown in Table III.

TABLE III

PARTICLE SIZE DISTRIBUTION

| Compound of Example 1 | ETOH | P 114 | P 12 | Part. Size (mg collected) per 20 actuations 1 μ | 2 μ | 4 μ* | MMAD** | % Recovery | Pressure PSI | mg at 1-8 μ | cum % 1-8 μ |
|---|---|---|---|---|---|---|---|---|---|---|---|
| 1.0 | 5.0 | 47 | 47 | 0.448 | 0.973 | 2.091 | 3.0 | 52.3 | 58.7 | 3.512 | 57.74 |
| 1.0 | 5.0 | 47 | 47 | 0.607 | 1.529 | 2.522 | 3.0 | 57.7 | -- | 4.658 | 60.73 |
| 1.0 | 5.0 | 47 | 47 | 0.667 | 1.532 | 2.481 | 2.8 | 53.97 | 60.2 | 4.680 | 66.20 |
| 1.0 | 10.0 | 44.5 | 44.5 | 0.498 | 1.243 | 1.871 | -- | 50.6 | 49.3 | 3.612 | 53.70 |
| 1.0 | 10.0 | 44.5 | 44.5 | 0.479 | 0.868 | 1.715 | 3.0 | 44.7 | -- | 3.062 | 57.26 |
| 1.0 | 10.0 | 35 | 53 | 0.503 | 1.201 | 2.283 | 3.5 | 56.1 | 56 | 3.987 | 55.41 |
| 1.0 | 10.0 | 25.0 | 75.0 | 0.499 | 1.167 | 1.611 | 4.0 | 56.0 | -- | 3.277 | 45.36 |
| 2.0 | 10.0 | 44.00 | 44.00 | 0.661 | 1.373 | 2.772 | 3.8 | 38.7 | -- | 4.806 | 46.71 |
| 2.5 | 10.0 | 43.75 | 43.75 | 0.514 | 1.380 | 3.121 | 3.8 | 34.6 | -- | 5.015 | 44.86 |
| 2.5 | 10.0 | 43.75 | 43.75 | 0.528 | 1.435 | 3.103 | 4.0 | 35.9 | -- | 5.066 | 44.36 |
| 2.5 | 10.0 | 43.75 | 43.75 | 0.638 | 1.106 | 2.744 | 3.8 | 33.8 | -- | 4.488 | 42.87 |
| 3.0 | 10.0 | 43.5 | 43.5 | 0.538 | 1.478 | 2.657 | 4.0 | 30.8 | -- | 4.673 | 40.15 |
| 3.5 | 10.0 | 43.25 | 43.25 | 0.583 | 1.236 | 3.546 | 4.5 | 34.6 | -- | 5.365 | 35.00 |
| 6.0 | 20.0 | 29.6 | 44.4 | 0.447 | 0.901 | 2.879 | 5.75 | 20.87 | 52.0 | 4.227 | 28.77 |
| 5.0 | 30.0 | 32.5 | 32.5 | 0.336 | 0.852 | 2.084 | 6.0 | 18.7 | 52 | 3.272 | 32.09 |
| 9.0 | 30.0 | 17 | 44 | 0.394 | 0.818 | 2.381 | 6.0 | 12.73 | 53.7 | 3.593 | 28.79 |

* Particle Size Limit = 4 μ to 8 μ

** MMAD = Mass Median Aerodynamic Diameter (particle size of 50 percentile of the cumulative percent by wt)

Based on large number of particle size distribution experiments, it has been observed that aerosol formulations containing 0.5% w/w to 2.5% w/w of the active compound to 5% w/w to 10% w/w ethanol are the most desirable formulations.

The aerosol formulations of the present invention provide drug delivery to the lungs within seconds and thereby effect rapid onset of activity. The active compounds of the aerosol formulations are 5-lipoxygenase inhibitors and leukotriene antagonists. The following describes the testing of efficacy of a 1% formulation of the compound of Example 1.

Test Method

Male Hartley guinea pigs (200-250 g) were immunized with ovalbumin, 2.7 mg/kg i.p., followed in 30 minutes by Salmonella typhosa lipopolysaccharide, 20 ug i.p. Thirteen to fifteen days after immunization, each guinea pig was anesthetized with sodium pentobarbital, 50 mg/kg i.p., and a jugular vein and the trachea were cannulated. The animal was mechanically ventilated (80 breaths/min. 2ml/breath). Needle electrodes were attached to the limbs for monitoring ECG and heart rate. Airway pressure was measured with a Validyne pressure transducer (± 20 cm $H_2O$ range) connected to the tracheal cannula. Airway pressure for each breath was recorded on a polygraph, while mean values over 6-second periods were printed on a digital terminal.

Each animal was treated with methapyrilene (2 mg/kg i.v.) and, 1 minute later, gallamine triethiodide (15 mg/kg i.v.), followed in 2 minutes by a priming dose of $LTD_4$ (0.8 ug/kg i.v.). A second dose of $LTD_4$ (0.8 ug/kg

i.v.) was administered 15 minutes later, followed immediately by indomethacin (10 mg/kg i.p.). Ten to fifteen minutes later, either 1% 2-(3-(1-hydroxyhexyl) phenoxymethyl)quinoline hydrochloride (compound of Example 1) or a vehicle consisting of 5% ethanol in Freons 114 and 12 (1:1), was administered by aerosol.

Delivery of the aerosol into the lungs was accomplished through the use of an oral adapter (i.e. a mouthpiece used for clinical administration of aerosols) attached via a Y-tube to the tracheal cannula. An aerosol bottle was fastened to the top of the adapter, and the aerosol particles were released (actuated) by pressing the dispenser downward while maintaining the adapter in a stable position. Aerosol actuations were given in sequence with every third inspiration produced by the ventilator, with a total of 25 actuations given to each animal. Each actuation contained approximately 0.1 mg of the active compound in the 1-8 micron range of particle size. Therefore, each animal treated with the formulation received approximately 5 mg of the active compound (or 15 mg/kg). Fifteen minutes after aerosol administration the animal was challenged with antigen (ovalbumin, 0.3 mg/kg i.v.).

All bronchoconstrictor responses to the second dose of $LTD_4$ and to antigen were quantified as the percent increase in airway pressure over the baseline value. The normalized response of each animal is expressed as the ratio of antigen-induced to $LTD_4$-induced bronchoconstriction.

Mean values and standard errors of these ratios in control and drug-treated groups were calculated and compared statistically by Student's t-test. The activity

of each drug is expressed as the percent inhibition of the mean control response.

Result

Animals (n=8) receiving the vehicle exhibited a normalized postchallenge increase in $P_{AW}$ of 1.28 ± 0.40, whereas animals (n=7) treated with the active compound containing aerosol formulation showed 53% inhibition, with a mean response of 0.60 ± 0.16.

WHAT IS CLAIMED IS:

1. A composition of matter to be dispensed from a drug delivery pressure device for the treatment of asthmatic conditions in a mammal comprising:

an effective amount of an antiasthmatic compound selected from

2-(3-(1-hydroxyhexyl)phenoxymethyl)quinoline hydrochloride,

2-(3-hexanoylphenoxymethyl)quinoline,

2-(3-(2-(2-hydroxy)heptyl)phenyloxymethyl)quinoline hydrochloride,

2-[3-(1-hydroxy-2-methylhexyl)phenoxymethyl]quinoline,

2-(3-(6-phenoxy-1-hydroxyhexyl)phenoxymethyl)quinoline,

2-[3-(6-phenoxy-1-hydroxy-1-methylhexyl)phenoxymethyl]quinoline,

2-[3-(1-hydroxy-2,2-dimethylhexyl)phenoxymethyl]quinoline,

2-(3-(1,2,2-trimethyl-1-hydroxyhexyl)phenoxymethyl)quinoline,

2-(3-(1-hydroxypentyl)phenoxymethyl)quinoline,

α-(3-2-quinolinylmethoxyphenyl)hexyl 2,2-dimethyl propanoate,

2-(3-(6-phenoxy-1,2,2-trimethyl-1-hydroxyhexyl)phenoxymethyl) quinoline,

2-(3-(1-hydroxy-6,6,6-trifluorohexyl)phenoxymethyl)quinoline, or

2-(3-(5,5,5-triflouro-1-methyl-1-hydroxypentyl)phenoxymethyl) quinoline or mixtures thereof;

dissolved in ethyl alcohol and admixed with a sufficient amount of a pharmaceutically acceptable propellant capable of effective transfer of said antiasthmatic

compound from said drug delivery pressure device to the oronasal passageways of a patient.

2. The composition of matter of claim 1 wherein said propellant is a mixture of two propellants.

3. The composition of claim 2 wherein said propellant mixture consists of dichlorodifluoromethane and dichlorotetrafluoroethane.

4. The composition of claim 2 wherein said propellant mixture consists of dichlorodifluoromethane and trichloromonofluoromethane.

5. The composition of any of claims 1-4 wherein the antiasthmatic compound is in the micronized form.

6. The composition of claim 5 including a pharmaceutically acceptable surfactant.

7. The composition of matter of claim 6 wherein said pharmaceutically acceptable surfactant is selected from oleyl alcohol, oleic acid, lecithin or the mixture of oleate esters of sorbitol and sorbitol anhydrides having the formula

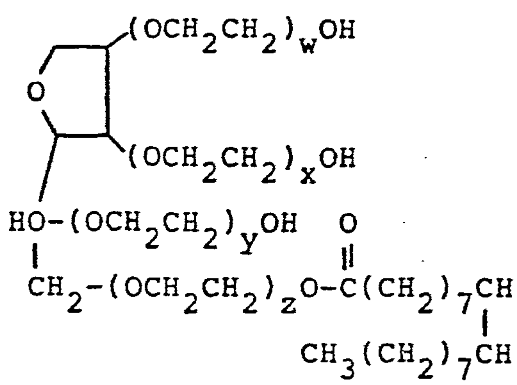

where w + x + y +z has an average value of 20.

8. The composition of matter according to any of claims 1-7 comprising

(a) 0.25% to 20% of the antiasthma active compound

(b) 2.5% to 30% ethyl alcohol; and

(c) 50% to 97.25% of a propellant mixture consisting of 15% to 50% of dichlorodifluoromethane and 30% to 50% dichlorotetrofluoroethane.

9. The composition of matter as in any of claims 1-7 comprising

(a) 0.25% to 10% of the antiasthma active compound

(b) 5% to 2-% ethyl alcohol; and

(c) 70% to 94.75% of a propellant mixture consisting of 15% to 50% of dichlorodifluoromethane and 30% to 50% dichlorotetrofluoroethane.

10. The composition of matter as in any of claims 1-7 comprising

(a) 0.5% to 2.5% of the antiasthma active compound

(b) 5% to 10% ethyl alcohol; and

(c) 87.5% to 94.5% of a propellant mixture consisting of equal parts of dichlorodifluoromethane and dichlorotetrofluoroethane.

11. The composition of matter as in any of claims 1-10 wherein said ethyl alcohol contains less than 5% w/w water.

12. The composition of matter as in any of claims 1-10 wherein said ethyl alcohol is anhydrous.

European Patent Office

# EUROPEAN SEARCH REPORT

0234500
Application number

EP 87 10 2321

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 110 405 (USV PHARMACEUTICAL CORP.) * page 1, lines 1-30; page 19, line 19, example 11; page 30, lines 1-18; page 29, table II, compound nos. 28-33; claims 1, 12 * & ZA - A - 83 8926 (Cat. D) | 1 | A 61 K 9/12<br>A 61 K 31/47 |
| | --- | | |
| P,A | EP-A-0 181 568 (USV PHARMACEUTICAL CORP.) * claims 1-10, 16, 17 * | 1 | |
| | --- | | |
| A | ROTE LISTE, 1985, page 27021, Editio Cantor, Frankfurt; * page 27 021 "Ventilat" * | 2-4 | |
| | --- | | TECHNICAL FIELDS SEARCHED (Int. Cl.4) |
| A | WO-A-8 501 876 (LOCKLEY SERVICES PTY. LTD.) * claims 8, 9 * | 2-4,6, 7 | A 61 K 31/00<br>A 61 K 9/00<br>A 61 K 47/00<br>C 07 D 215/00<br>C 07 C 143/00 |
| | --- | | |
| A | KIRK-OTHMER "Encyclopedia of chemical technology", 3rd edition, vol. 14, 1981, page 266, John Wiley & Sons, New York, US; * page 266 "Pharmaceuticals" * | 6,7 | |
| | --- -/- | | |

The present search report has been drawn up for all claims

| Place of search | Date of completion of the search | Examiner |
|---|---|---|
| BERLIN | 05-05-1987 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

Application number 0234500

EP 87 10 2321

Page 2

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| A | KIRK-OTHMER "Encyclopedia of chemical technology", 3rd edition, vol. 14, 1981, pages 370-371, John Wiley & Sons, New York, US; * pages 370-371 "Ethoxylated Anhydrosorbitol Esters" * | 6,7 | |
| | ----- | | |
| | | | TECHNICAL FIELDS SEARCHED (Int. Cl.4) |

The present search report has been drawn up for all claims

| Place of search | Date of completion of the search | Examiner |
|---|---|---|
| BERLIN | 05-05-1987 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document